# EUROPEAN PATENT SPECIFICATION

(11) **EP 4 227 438 B1**
(45) Date of publication and mention of the grant of the patent: **25.12.2024**
(21) Application number: 22156365.3
(22) Date of filing: 11.02.2022
(51) Int. Cl.: C25B 1/04, C25B 1/34, C25B 9/70, C25B 15/023

(54) **METHOD FOR OPERATING A PLURALITY OF ELECTROLYSER-STACKS, AND ELECTROLYSER-STACK OPERATION SYSTEM**
VERFAHREN ZUM BETRIEB EINER VIELZAHL VON ELEKTROLYSEURSTAPELN, UND ELEKTROLYSEURSTAPELN-BETRIEBSSYSTEM
PROCÉDÉ DE FONCTIONNEMENT D'UNE PLURALITÉ D'EMPILEMENTS D'ÉLECTROLYSEURS, ET SYSTÈME DE FONCTIONNEMENT POUR UNE PLURALITÉ D'EMPILEMENTS D'ÉLECTROLYSEUR

(43) Date of publication of application: 16.08.2023
(73) Proprietor: ABB SCHWEIZ AG, 5400 Baden (CH)
(72) Inventor: BISKOPING, Matthias, 69493 Hirschberg (DE); PRIMAS, Bernhard, 68165 Mannheim (DE); LEAL-AYALA, Andres, 5408 Ennetbaden (CH); GUTERMUTH, Georg, 69115 Heidelberg (DE); KOENIG, Kai, 69190 Walldorf (DE); THORBURN, Stefan, 725 97 Västerås (SE); CHARTOUNI, Daniel, 5430 Wettingen (CH)
(74) Representative: Maiwald GmbH

(56) References cited:
- WO-A1-2014/016057
- FR-A1- 3 014 452
- JP-A- 2021 070 849
- LEE JASON K. ET AL: "Critical Current Density as a Performance Indicator for Gas-Evolving Electrochemical Devices", CELL REPORTS PHYSICAL SCIENCE, vol. 1, no. 8, 26 August 2020 (2020-08-26), pages 100147, XP055942461, ISSN: 2666-3864, DOI: 10.1016/j.xcrp.2020.100147

## Description

The present invention relates to a method for operating a plurality of electrolyser-stacks, and an electrolyser-stacks operation device, see claims 1 and 15.

A number of electrolysis processes have industrial applications. For example, the electrolysis of water provides a source of hydrogen and oxygen. Alternatively, the electrolysis of water containing sodium chloride can provide a source of hydrogen, chlorine, and sodium hydroxide. Water electrolysis processes fundamentally involve the splitting of water into its constituents although other chemistries also utilizing the electrolysis of water are certainly possible.

In a water-electrolyser water is split into its components: hydrogen gas and oxygen gas. An electrolyser essentially comprises a cathode-electrode, an anode-electrode, and an electrolyte. Hydrogen is produced at the cathode via the Hydrogen Evolution Reaction (HER). Oxygen is produced at the anode via the Oxygen Evolution Reaction (OER).

FR 3 014 452 A1 (describing the preamble of claim 1), provides, according to its abstract, an electrolysis system comprises an electrolyser which is provided with an electrolytic membrane having an oxygen outlet and a hydrogen outlet, and a separator outlet used for carrying out separation of liquid/gas. A measuring device is connected to the outlet of the electrolyser. The measuring device is connected to an inlet of the separator outlet for measuring the content of hydrogen and/or oxygen in the fluid.

### Description

For operation of a plurality of electrolyser-stacks it is useful to control a correct performance of each of the electrolyser-stacks and/or each cell of the electrolyser-stacks. The invention relates to the field of electrolysis technology and relates to an improved method for operating an electrolysis cell/stack mainly in respect of monitoring failure situations.

Considering a large scale, multi-megawatt hydrogen production facility consisting of dozens of water electrolysers, it is necessary to have an efficient method to guarantee hydrogen production with enhanced purity and reduced safety risks.

When such an electrolyser park, including a plurality of electrolyser-stacks, is operating at full capacity, oxygen and hydrogen separation in the electrolysis cell can be optimal. However, when a decrease in the availability of renewable energy forces the control system to reduce the operating capacity of one or more electrolysers, pressure gradients in the electrolysis cells can lead to hydrogen diffusion through the membrane to the oxygen side of the cell, or oxygen diffusion to the hydrogen side of the cell. Hydrogen diffusion to the oxygen half-cell can create an explosive atmosphere if a safety limit of about 2% hydrogen in oxygen is exceeded, while oxygen diffusion into the hydrogen half-cell can reduce the purity of the hydrogen, increasing energy consumption for purification purposes in subsequent process steps, reducing an overall efficiency of the system.

A use of physical hydrogen and oxygen sensors to monitor the situation at every electrolyser-stack of the plurality of electrolyser-stacks in a multi MW-facility can end up with the installation of hundreds of sensors. A process requiring hundreds of sensors can be unreliable and can have very high maintenance costs associated with ensuring the accuracy of every sensor.

Accordingly, the present invention defines a method for operating a plurality of electrolyser-stacks, and an electrolyser-stack operation device according to claims 1 and 15.

Advantageous modifications of the invention are stated in the dependent claims.

In this entire description of the invention, the sequence of procedural steps is presented in such a way that the process is easily comprehensible.

To achieve these and other advantages and in accordance with the purpose of the invention, there is provided a method for operating a plurality of electrolyser-stacks according to claim 1, wherein each of the plurality of electrolyser-stacks are configured to be provided with water and electrical energy to produce at least a first reaction gas electrochemically at a first electrode type of each of the electrolyser-stacks, and wherein the first reaction gas produced by each of the plurality of electrolyser-stacks is merged into a first gas stream, including the following steps: in one step a concentration of impurities, which is originated by a second reaction gas electrochemically produced at a second electrode type of each of the electrolyser-stacks is determined, within the first gas stream. In another step a trigger signal is generated if the concentration of the impurities of the second reaction gas within the merged first reaction gas exceeds a specific second reaction gas level. In another step at least one electrolyser-stack out of the plurality of electrolyser-stacks is identified, which is low performing in respect to excessively feeding second reaction gas impurities into the first gas stream, by measuring a current density of at least one electrolyser-stack of the plurality of electrolyser-stacks, if the trigger signal is generated.

An electrolyser-stack can be a stack of electrolyser-cells, which are stacked and are electrically coupled to each other in respect to electrical power provided to the stack, such that each electrolyser-cell, which can include an anode-electrode and a cathode-electrode, wherein the anode-electrode and the cathode-electrode being different electrode types are arranged at opposite sides of a membrane, which can act as an electrolyte. An amount of electrolytic generated gas at the anode-electrode and/or the cathode-electrode can be collected from each electrolyser-cell to build an anode-flow of gas for the anode-electrodes as well as a cathode-flow of gas at the cathode-electrodes of each electrolyser-stack.

An electrolyser-stack can produce a first reaction gas electrochemically at the anode-electrodes and different first reaction gas electrochemically at the cathode-electrodes of the electrolyser-stack at the same time. The first reaction gas can be a reaction product formed by an electrolyse reaction at a respective type of electrode.

The trigger signal can be an alarm or an alarm signal, which is generated if a concentration, which is e.g. determined by a gas sensitive sensor, of the second reaction gas exceeds a specific limit.

A flow of first reaction gas of a plurality of individual electrolyser-stack can be merged by a system of pipes fluidly connected to each electrolyser-stack, wherein the pipes are connected accordingly. The merged first gas stream can be collected using a tank, which is connected to the system of pipes accordingly.

According to an aspect, the identification of a low performing electrolyser-stack by measuring a current density of an electrolyser-stack can alternatively or additionally be done by measuring a current density of a subsystem of the electrolyser-stack, as for instance a part of electrolyser-stack-cells and/or individual electrolyser-stack-cell.

According to an aspect, the measurement of the current density of the at least one electrolyser-stack can be triggered by the trigger signal and/or the measurement of the current density can be continuously monitored and values of the measurement can be analyzed, if triggered by the trigger signal.

Using other words, the method provides a solution that instead of monitoring the situation of every electrolysis-stack of the plurality of electrolysers-stack with a plurality of gas-sensitive sensors, at least a sensor can be located within the first gas stream and/or in a storage tank for the merged and/or collected reaction gas, as for instance hydrogen gas and oxygen gas, produced by several electrolyser-stacks. If an impurity level, respectively a concentration of the second reaction gas, in the first gas stream and/or the storage tank is within the required safety and quality levels, e.g. less than 2% hydrogen gas in oxygen gas, it is considered that all electrolyser-stacks feeding the storage tank are operating correctly. If a threshold value of impurities of the second reaction gas within the first reaction case is exceeded, an alarm signal can be triggered, and an algorithm can be used to identify the faulty electrolysis-stack(s). Once the alarm signal is triggered, a control system, as an electrolyser-stack operation device, can determine a current density generated by individual electrolysis-stacks, which feeds the storage tank. The determined values of the current density can be used as soft sensors or virtual sensors to determine an impurity concentration, since for each of these electrolyser-stacks, a calibration curve can be set up upfront, which relates the determined current density of the individual electrolyser-stack to a concentration of hydrogen gas within the produced oxygen gas. The hydrogen gas can be originated by a diffusion process from a cathode electrode of a cell of the electrolyser-stacks to the respective anode electrode of an oxygen side of the cell of the electrolyser-stack. That means an anodic hydrogen content can be determined by using the calibration curve.

Using an algorithm for the determination of the current density of individual electrolysers-stacks, by comparing an actual current density with the calibration curve, can be used to identify a faulty or low performing electrolyser-stack and electrical power provided to that low performing electrolyser-stack can be bypassed or disconnected.

As a result, the low performing electrolyser-stack can be serviced, such that the hydrogen gas concentration within a produced oxygen gas can be kept below the safety limit. In addition, for the production of hydrogen gas a concentration of oxygen gas within a produced and collected hydrogen gas can be kept below a required quality level.

Advantageously, based on the pre-existing calibration curves of the electrolyser-stacks, and amount of anodic hydrogen and cathodic oxygen can be determined for each electrolyser-stack in a real-time manner, without disconnecting individual electrolyser-stacks. By measuring an impurity concentration of anodic hydrogen and/or cathodic oxygen within the merged first gas stream within a pipe and/or a tank an early detection system can be set up. As a result, there can be provided a redundancy to physical sensors located in the collecting tanks, were hydrogen gas and oxygen gas is stored, thereby increasing a reliability of the system.

That means, that instead of monitoring the impurity concentration of each electrolysis stack using individual sensors, it is possible to locate at least one sensor within the merged first gas stream and/or a storage tank, where the first gas stream, as the hydrogen gas stream and/or oxygen gas stream produced by several electrolyser-stacks is collected. If the impurity levels determined by the sensor exceeds a threshold value, an alarm signal or a trigger signal can be generated and an algorithm can be used to identify the faulty electrolysis stacks by measuring the current density of each electrolysis stack, using the determined current density values as a soft sensor.

According to an aspect, the first reaction gas electrochemically produced at the first electrode type is oxygen and the second reaction gas electrochemically produced at the second electrode type is hydrogen. Alternatively or additionally the first reaction gas electrochemically produced at the first electrode type is hydrogen and the second reaction gas electrochemically produced at the second electrode type is oxygen.

That means, that oxygen gas as a first reaction gas can be produced at an anode-electrode and/or hydrogen as a first reaction gas can be produced at a cathode electrode.

That means, that with the method described above a hydrogen content in an oxygen stream can be monitored in this way, as well as an oxygen content in a hydrogen stream, such that, if a corresponding impurity level threshold value is reached, corrective actions can be taken. By this, a purity level of oxygen gas within hydrogen gas can be enhanced and by determining a hydrogen level within oxygen gas safety risks can be reduced by this soft sensing.

According to an aspect, the identification of the at least one low performing electrolyser-stack out of the plurality of electrolyser-stacks, is done by comparing the measured current density of the at least one electrolyser-stack with a critical current density. Particularly the at least one low performing electrolyser-stack can be identified if the measured current density of that electrolyser-stack is below the critical current density.

That is because a typical relationship between the current density of the electrolysers-stacks and the impurity level is reverse proportional, which can be shown in particular by means of a polarization curve, at least as an approximation.

According to an aspect, specific second reaction gas level is a hydrogen reaction gas level if the first reaction gas is oxygen. If the specific second reaction gas level is an oxygen reaction gas level the first reaction gas is hydrogen. Particularly the hydrogen reaction gas level and the oxygen reaction gas level can be different.

According to an aspect, a current density of each of several of the plurality of electrolyser-stacks are measured and each of the measured current densities are compared with an individual critical current density, which is assigned to each of the several electrolyser-stacks, to identify a single low performing electrolyser-stack with a measured current density, which is lowest below its individual critical current density, to identify the low performing electrolyser-stack.

In this respect, the identified low performing electrolyser-stack can be seen as the electrolyser-stack producing a highest percentage of impurity, because its current density is most below its individual critical current density.

Using other words, that electrolyser-stack should be identified which is worst operating in respect of impurities production and particularly this identified electrolyser-stack can be disabled or shutdown.

According to an aspect, the critical current density is updated during the operation of the plurality of electrolyser-stacks to account for degradation of the electrolyser-stacks.

During operation, the membrane can age or degrade, eventually leading to a higher diffusivity of the gases from one electrolytic half-cell of the electrolyser-stacks to the other. As a result of this, a calibration curve of the electrolyser-stack can be shifted upwards and/or a critical current density of the electrolyser-stack can be changed. That means, that with time, e.g. a 2% threshold value of impurities can result from higher current densities, e.g. changing from 1.0 A/cm^2 to 1.2 A/cm^2.

If a current density is determined at each electrolyser-stack it enables to individual update the calibration curve, respectively the critical current density, as the electrolytic cells age.

If for instance, a plurality of electrolyser-stack is in production for two years the calibration curve and/or the critical current density of all or individual electrolyser-stacks can be updated. For this update, at least time by time or on a regular base, current density and voltage of the individual electrolyser-stacks are measured, particularly to update the calibration curve and/or the critical current density. Because of operating the electrolyser-stacks at different operation points, caused by an amount of electrical energy which is provided to the plurality of electrolyser-stacks over time, a calibration curve can be updated by storing this values. Alternatively or additionally, the production load can be distributed between some of the electrolyser-stacks of the plurality of electrolyser-stacks to operate the electrolyser-stack at different set points and by this the calibration curve and/or the critical current density can be determined. Using other words, a set point for operation of an electrolyser-stack can be swept over time.

According to an aspect, the individual critical current density for each of the several electrolyser-stacks is updated during the operation of these electrolyser-stacks to account for degradation of these electrolyser-stacks.

According to an aspect, the updated critical current density and/or the updated individual critical current density of the electrolyser-stacks is determined by a linear relationship between operating hours of at least one electrolyser-stack and the critical current density of that electrolyser-stack.

Using other words, on a base of an aging curve for the electrolyser-stack respectively of the calibration curve, a shift of the calibration curve over time can be used to determine the critical current density, particularly of an individual electrolyser-stack.

According to an aspect, the updated critical current density and/or the updated individual critical current density of the electrolyser-stack is determined during operation of the plurality of electrolyser-stacks by operating at least some electrolyser-stacks of the plurality of electrolyser-stacks at different set points. The current density determines how much Oxygen and Hydrogen is produced at that an individual operating point. That means with other words, by comparing that operating point with a given calibration curve, which can be updated during operation, a crossover, respectively a diffusion of one gas to the other side of a membrane of the electrolyser-stack can be determined.

By shifting or modifying the operational point of the electrolyser-stack, the production load of individual electrolyser-stacks of the plurality of electrolyser stacks can be shifted. A set point, respectively an operation point, of an electrolyser-stack can be defined by the operating conditions, mainly determined by a voltage and current provided to the electrolyser-stack, at which the electrolyser-stack is operating for producing a specified first reaction gas.

Each electrolyser-stack can be configured for determining DC and/or AC current and DC and/or AC voltage measurements. Particularly these measurements can be used for Electrochemical Impedance Spectroscopy analysis, which finally allows to determine a degradation of the analysed electrolyser-stack, to be used as an input and/or to update a calibration curve, based on the result of the Electrochemical Impedance Spectroscopy analysis, after a period of hours of operation.

According to an aspect, the updated critical current density and/or the updated individual critical current density of the electrolyser-stacks is determined by storing values of voltage and current density of the electrolyser-stacks during operation of the plurality of electrolyser-stacks at different set points within a period of operation time.

According to an aspect, the updated critical current density and/or the updated individual critical current density of the electrolyser-stacks is determined by impedance spectroscopy measurement of at least several electrolyser-stacks of the plurality of electrolyser stacks.

Advantageously, adapting the set point for operating the electrolyser-stacks can be done by the result of the impedance spectroscopy measurements, particularly for determining a Polarization Curve at different Frequencies (Impedance Spectroscopy) or at least at frequencies that are excited by the electric power supply of the electrolyser-stacks for operation. Particularly low frequency variations can even be triggered by the overall control system of a plant with the plurality of electrolyser-stacks via the power supply.

According to an aspect, the impedance spectroscopy measurement is performed by using harmonics injected by the electrical power provided to the plurality of electrolyser-stacks.

According to an aspect, the identified low performing electrolyser-stack is disconnected from its provided electrical energy.

For this the electrical energy can be bypassed if the plurality of electrolyser-stacks are electrically connected in series or disconnected, e.g. by opening a switch.

According to an aspect, the above described steps of identifying a single electrolyser-stack with the lowest current density and disconnecting it from its provided electrical energy can be repeated until the trigger signal is no longer generated. Advantageously, using this algorithm to identify electrolyser-stacks to be disconnected can keep a production rate of the first reaction gas with a multitude of electrolyser-stacks at a high level, by still providing high quality and safety standards.

According to an aspect, the waiting time after an identified electrolyser-stack is disconnected can be introduced, which is long enough that the system of electrolyser-stacks can approach a new equilibrium or steady state, before the new measurement of the impurity concentration is performed.

According to an aspect, the concentration of impurities of the second reaction gas is determined by a gas sensitive sensor for generating the trigger signal.

Such a gas sensitive sensor can be located within a tank collecting the merged first gas stream and/or the gas sensitive sensor can be located within a pipe carrying the merged first gas stream before entering the tank.

According to an aspect, the gas sensitive sensor is located for sensing within the first gas stream. Alternatively or additionally the gas sensitive sensor is located for sensing within a tank, wherein the tank is configured in respect to the plurality of electrolyser-stacks for collecting the first reaction gas of the first gas stream.

An electrolyser-stack operation device in accordance with the present invention is defined in claim 1, the device including:
- a current density measurement input, which is configured to receive current density measurement values of each of a plurality of electrolyser-stacks; and
- a switch control device, which is configured to be signally coupled to switches of each of the plurality of electrolyser-stacks, for disconnecting any electrolyser-stack of the plurality of electrolyser-stacks from the provided electrical energy; and
- a trigger signal interface and/or an interface for a gas sensitive sensor; and
- a control device, particularly including a computer, which is signally coupled with the current density measurement input and to the switch control device and to the first signal interface and/or the interface for the gas sensitive sensor. The control device can be configured to perform a method for operating a plurality of electrolyser-stacks as described above.

### Brief description of the drawings

The accompanying drawings, which are included to provide a further understanding of the invention and are incorporated in and constitute a part of this application, illustrate embodiments of the invention and together with the description serve to explain the principle of the invention. The drawing displays:
Figure 1 a calibration curve; and
Figure 2 a system for electrochemical production of a first reaction gas.

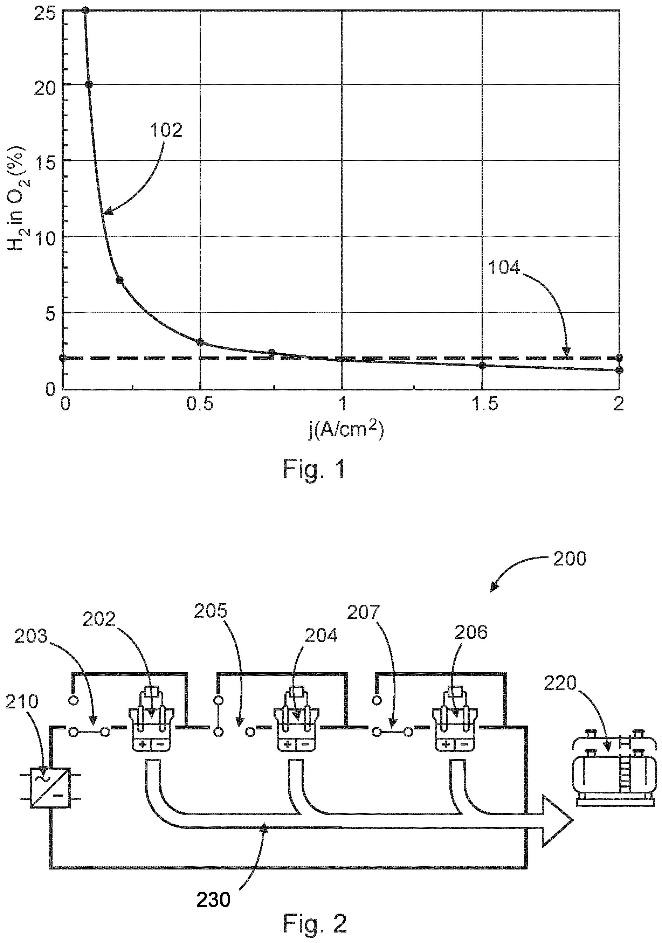

Figure 1 schematically sketches a calibration curve as a diagram showing the relationship 102 between the current density j measured in A/cm² and a concentration of hydrogen gas in oxygen gas measured in percent. Within the diagram a limit curve 104 is drawn for identification of the critical current density j, wherein the critical current density can be determined where the curve 102 and the limit curve 104 cross each other.

Figure 2 schematically sketches a system for electrochemical production of a first reaction gas, wherein the first reaction gas can be oxygen gas or hydrogen gas. The system includes an electrical power supply 210 a plurality of electrolyser-stacks 202, 204, 206 which are configured to be electrically coupled to the power supply 210 in series. Each of the plurality of electrolyser-stacks 202, 204 and 206 can be disconnected from the electrical power supply 210 by a switch 203, 205, 207 for bypassing the electrical current to the next electrolyser-stack 202, 204, 206. A first reaction gas which can be electrochemically produced by each of the electrolyser-stacks 202, 204, 206 are merged into a first gas stream 230, e.g. by a system of pipes, which are fluidly coupled to each of the electrolyser-stacks 202, 204, 206, and where the first gas stream 230 can be fed into a tank 220, e.g. by means of the system of pipes.

A gas sensitive sensor can be located within the first gas stream 230 and/or the tank 220. The physical gas sensitive sensor can be configured to monitor an amount of oxygen gas in the hydrogen stream if the first gas stream 230 mainly includes hydrogen gas. Alternatively or additionally, if the first gas stream 230 mainly includes oxygen gas the gas sensitive sensor can be configured to monitor an amount of hydrogen gas in the oxygen gas stream. For the case that as well hydrogen gas and oxygen gas is produced by the electrolyser-stacks, a second pipe system including a second tank, which are not shown here, is connected to the electrolyser-stacks, which are configured to produce oxygen gas as well as hydrogen gas.

If such the gas sensitive sensor determines an impurity concentration exceeding a threshold value, of e.g. 2% oxygen in hydrogen, the system is configured, e.g. by an electrolyser-stack operation device, not shown here, to generate a trigger signal or an alarm signal. If the trigger signal is generated the electrolyser-stack operation device can identify an optimal way to reduce the amount of oxygen impurity within the produced hydrogen gas, while minimizing the impact on a hydrogen production rate, e.g. by bypassing the electrolyser stack 204, while maintaining the electrolyser-stacks 202 and 206 in operation as closer described by an example below.

The three electrolyser-stacks 202, 204, 206 can be configured to produce hydrogen at different production rates and different oxygen impurity levels. The hydrogen gas thus produced by all three stacks 202, 204, 206 can be directed to be collected in one common storage tank 220. As an example, stack 202 has a hydrogen gas production rate of 300 m³/h with 3% of oxygen impurity; stack 204 produces hydrogen gas at a rate of 200 m³/h with 5% of oxygen impurity; and stack 206 produces 800 m³/h of hydrogen containing 1% of oxygen impurity. These hydrogen gas flow, when collected in the storage tank, which is equipped with a physical oxygen sensor, has an overall oxygen gas impurity content of 2.07%, which is above a 2% threshold value 104.

As the trigger signal is generated, the electrolyser-stack operation device is set up to determine and/or to analyse the current densities of each of the individual stacks 202, 204, 206, comparing the real-time values of the current density with the limit value of the calibration curve 102. The electrolyser-stack operation device identifies that stacks 202 and 204 are producing hydrogen with an oxygen content above the allowed value of 2%, but the electrolyser-stack operation device also determines, e.g. by disconnecting the electrolyser-stack 204, with the lowest current density, that by shutting down the electrolyser-stack 204, the overall oxygen level in the storage tank is brought down from 2.07% to 1.54%, avoiding the need to shut down the electrolyser-stack 202.

Therefore, the electrolyser-stack operation device can operate the switches 203, 205, 207 as shown in Fig 2, by bypassing electrolyser-stack 204, while maintaining electrolyser stacks 202 and 206 in operation, minimizing production rate reduction, while guaranteeing the quality and safety standards.

Similarly, the hydrogen content in the oxygen streams can also be monitored in this way, so that when the threshold value is reached, corresponding corrective actions can be taken.

## Claims

1. A method for operating a plurality of electrolyser-stacks (202, 204, 206), wherein each of the plurality of electrolyser-stacks (202, 204, 206) are configured to be provided with water and electrical energy to produce at least a first reaction gas electrochemically at a first electrode type of each of the electrolyser-stacks (202, 204, 206), and wherein the first reaction gas produced by each of the plurality of electrolyser-stacks (202, 204, 206) are merged into a first gas stream (230), comprising:
determining a concentration of impurities, which is originated by a second reaction gas electrochemically produced at a second electrode type of each of the electrolyser-stacks, within the first gas stream (230);
the method being **characterising by** the following steps:
generating a trigger signal if the concentration of the impurities of the second reaction gas within the merged first reaction gas (230) exceeds a specific second reaction gas level (104);
identifying at least one electrolyser-stack out of the plurality of electrolyser-stacks (202, 204, 206), which is low performing in respect to excessively feeding second reaction gas impurities into the first gas stream (230), by measuring a current density of at least one electrolyser-stack of the plurality of electrolyser-stacks (202, 204, 206), if the trigger signal is generated.

2. The method according to claim 1, wherein the first reaction gas electrochemically produced at the first electrode type is oxygen and the second reaction gas electrochemically produced at the second electrode type is hydrogen; or wherein the first reaction gas electrochemically produced at the first electrode type is hydrogen and the second reaction gas electrochemically produced at the second electrode type is oxygen.

3. The method according to any of the preceding claims, wherein the identification of the at least one low performing electrolyser-stack out of the plurality of electrolyser-stacks (202, 204, 206), is done by comparing the measured current density of the at least one electrolyser-stack with a critical current density (104); and particularly identify the at least one low performing electrolyser-stack if the measured current density is below the critical current density (104).

4. The method according to claim 2 or 3, wherein the specific second reaction gas level is a hydrogen reaction gas level if the first reaction gas is oxygen; and wherein the specific second reaction gas level is an oxygen reaction gas level if the first reaction gas is hydrogen; and wherein particularly the hydrogen reaction gas level and the oxygen reaction gas level are different.

5. The method according any of claim 3 or 4, wherein a current density of each of several of the plurality of electrolyser-stacks (202, 204, 206) are measured and each of the measured current densities are compared with an individual critical current density, which is assigned to each of the several electrolyser-stacks, to identify a single low performing electrolyser-stack with a measured current density, which is lowest below its individual critical current density, to identify the low performing electrolyser-stack.

6. The method according to any one of claims 3 or 5, wherein the critical current density is updated during the operation of the plurality of electrolyser-stacks (202, 204, 206) to account for degradation of the electrolyser-stacks.

7. The method according to claim 5, wherein the individual critical current density for each of the several electrolyser-stacks is updated during the operation of these electrolyser-stacks to account for degradation of these electrolyser-stacks.

8. The method according to claim 6 or 7, wherein the updated critical current density and/or the updated individual critical current density of the electrolyser-stacks is determined during operation of the plurality of electrolyser-stacks by operating at least some electrolyser-stacks of the plurality of electrolyser-stacks at different set points.

9. The method according to any of claims 6 to 8, wherein the updated critical current density and/or the updated individual critical current density of the electrolyser-stacks is determined by impedance spectroscopy measurement of at least several electrolyser-stacks of the plurality of electrolyser stacks (202, 204, 206).

10. The method according to claim 9, wherein the impedance spectroscopy measurement is performed by using harmonics injected by the electrical power provided to the plurality of electrolyser-stacks.

11. The method according to any of the preceding claims, wherein the identified low performing electrolyser-stack is disconnected from its provided electrical energy.

12. The method according to claim 5 and claim 11, which is repeated until the trigger signal is no longer generated.

13. The method according to any of the preceding claims, wherein the concentration of impurities of the second reaction gas is determined by a gas sensitive sensor for generating the trigger signal.

14. The method according to claim 13, wherein the gas sensitive sensor is located for sensing within the first gas stream (230); or wherein the gas sensitive sensor is located for sensing within a tank (220), wherein the tank (220) is configured in respect to the plurality of electrolyser-stacks (202, 204, 206) for collecting the first reaction gas of the first gas stream (230).

15. An electrolyser-stack operation device, comprising:
a current density measurement input, which is configured to receive current density measurement values of each of a plurality of electrolyser-stacks (202, 204, 206);
a switch control device, which is configured to be signally coupled to switches (203, 205, 207) of each of the plurality of electrolyser-stacks (202, 204, 206), for disconnecting any electrolyser-stack of the plurality of electrolyser-stacks (202, 204, 206) from the provided electrical energy;
a trigger signal interface and/or an interface for a gas sensitive sensor; and
a control device, particularly including a computer, which is signally coupled with the current density measurement input; and signally coupled to the switch control device; and signally coupled to the first signal interface and/or the interface for the gas sensitive sensor; and wherein the control device is configured to perform a method according to claim 1 to 14.

## Patentansprüche

1. Verfahren zum Betreiben einer Vielzahl von Elektrolyseur-Stacks (202, 204, 206), wobei jeder der Vielzahl von Elektrolyseur-Stacks (202, 204, 206) so konfiguriert ist, dass er mit Wasser und elektrischer Energie versorgt wird, um mindestens ein erstes Reaktionsgas elektrochemisch an einem ersten Elektrodentyp jedes der Elektrolyseur-Stacks (202, 204, 206) zu erzeugen, und wobei das erste Reaktionsgas, das von jedem der Vielzahl der Elektrolyseur-Stacks (202, 204, 206) erzeugt wird, zu einem ersten Gasstrom (230) zusammengeführt wird, umfassend:
Bestimmen einer Konzentration von Verunreinigungen, die von einem zweiten Reaktionsgas stammen, welches an einem zweiten Elektrodentyp jedes der Elektrolyseur-Stacks elektrochemisch erzeugt wird, in dem ersten Gasstrom (230);
wobei das Verfahren durch die folgenden Schritte gekennzeichnet ist:
Erzeugen eines Trigger-Signals, wenn die Konzentration der Verunreinigungen des zweiten Reaktionsgases in dem zusammengeführten ersten Reaktionsgas (230) eine bestimmte zweiten Reaktionsgasschwelle (104) überschreitet;
Identifizieren mindestens eines Elektrolyseur-Stacks aus der Vielzahl von Elektrolyseur-Stacks (202, 204, 206), der eine geringe Performance in Bezug auf die übermäßige Zufuhr von Verunreinigungen des zweiten Reaktionsgases in den ersten Gasstrom (230) aufweist, durch Messen einer Stromdichte mindestens eines Elektrolyseur-Stacks aus der Vielzahl von Elektrolyseur-Stacks (202, 204, 206), wenn das Trigger-Signal erzeugt wird.

2. Verfahren gemäß Anspruch 1, wobei das erste Reaktionsgas, das elektrochemisch an dem ersten Elektrodentyp erzeugt wird, Sauerstoff ist und das zweite Reaktionsgas, das elektrochemisch an dem zweiten Elektrodentyp erzeugt wird, Wasserstoff ist; oder wobei das erste Reaktionsgas, das elektrochemisch an dem ersten Elektrodentyp erzeugt wird, Wasserstoff ist und das zweite Reaktionsgas, das elektrochemisch an dem zweiten Elektrodentyp erzeugt wird, Sauerstoff ist.

3. Das Verfahren gemäß einem der vorstehenden Ansprüche, wobei die Identifizierung des mindestens einen Elektrolyseur-Stacks mit geringer Performance aus der Vielzahl von Elektrolyseur-Stacks (202, 204, 206) durch Vergleichen der gemessenen Stromdichte des mindestens einen Elektrolyseur-Stacks mit einer kritischen Stromdichte (104) erfolgt; und insbesondere den mindestens einen Elektrolyseur-Stack mit geringer Leistung zu identifizieren, wenn die gemessene Stromdichte unter der kritischen Stromdichte (104) liegt.

4. Verfahren gemäß Anspruch 2 oder 3, wobei die spezifische zweite Reaktionsgasschwelle ein Wasserstoff-Reaktionsgasschwelle ist, wenn das erste Reaktionsgas Sauerstoff ist; und wobei die spezifische zweite Reaktionsgasschwelle ein Sauerstoff-Reaktionsgasschwelle ist, wenn das erste Reaktionsgas Wasserstoff ist; und wobei insbesondere die Wasserstoff-Reaktionsgasschwelle und die Sauerstoff-Reaktionsgasschwelle unterschiedlich sind.

5. Verfahren gemäß einem der Ansprüche 3 oder 4, wobei eine Stromdichte von jedem von mehreren der Vielzahl von Elektrolyseur-Stacks (202, 204, 206) gemessen wird und jede der gemessenen Stromdichten mit einer individuellen kritischen Stromdichte verglichen wird, die jedem der mehreren Elektrolyseur-Stacks zugeordnet ist, um einen einzelnen Elektrolyseur-Stack mit geringer Performance zu identifizieren, dessen gemessene Stromdichte am niedrigsten unterhalb seiner individuellen kritischen Stromdichte liegt, um den Elektrolyseur-Stack mit geringer Performance zu identifizieren.

6. Verfahren gemäß einem der Ansprüche 3 oder 5, wobei die kritische Stromdichte während des Betriebs der Vielzahl von Elektrolyseur-Stacks (202, 204, 206) aktualisiert wird, um eine Degradation der Elektrolyseur-Stacks zu berücksichtigen.

7. Das Verfahren gemäß Anspruch 5, wobei die individuelle kritische Stromdichte für jeden der mehreren Elektrolyseur-Stacks während des Betriebs dieser Elektrolyseur-Stacks aktualisiert wird, um die Degradation dieser Elektrolyseur-Stacks zu berücksichtigen.

8. Verfahren gemäß Anspruch 6 oder 7, wobei die aktualisierte kritische Stromdichte und/oder die aktualisierte individuelle kritische Stromdichte der Elektrolyseur-Stacks während des Betriebs der Vielzahl von Elektrolyseur-Stacks durch Betreiben von mindestens einigen Elektrolyseur-Stacks der Vielzahl von Elektrolyseur-Stacks bei unterschiedlichen Sollwerten bestimmt wird.

9. Verfahren gemäß einem der Ansprüche 6 bis 8, wobei die aktualisierte kritische Stromdichte und/oder die aktualisierte individuelle kritische Stromdichte der Elektrolyseur-Stacks durch Impedanzspektroskopie-Messung von mindestens mehreren Elektrolyseur-Stacks der Vielzahl von Elektrolyseur-Stacks (202, 204, 206) bestimmt wird.

10. Verfahren gemäß Anspruch 9, wobei die Impedanzspektroskopie-Messung unter Verwendung von Oberwellen durchgeführt wird, die mit der elektrischen Leistung, die der Vielzahl von Elektrolyseur-Stacks zugeführt wird, eingespeist werden.

11. Das Verfahren gemäß einem der vorstehenden Ansprüche, bei dem der identifizierte Elektrolyseur-Stack mit geringer Performance von seiner elektrischen Energie-Versorgung getrennt wird.

12. Das Verfahren gemäß Anspruch 5 und Anspruch 11, das wiederholt wird, bis das Trigger-Signal nicht mehr erzeugt wird.

13. Verfahren gemäß einem der vorhergehenden Ansprüche, wobei die Konzentration von Verunreinigungen des zweiten Reaktionsgases durch einen gassensitiven Sensor zur Erzeugung des Trigger-Signals bestimmt wird.

14. Verfahren gemäß Anspruch 13, wobei der gassensitive Sensor zum Erfassen innerhalb des ersten Gasstroms (230) angeordnet ist; oder wobei der gassensitive Sensor zum Erfassen innerhalb eines Tanks (220) angeordnet ist, wobei der Tank (220) in Bezug auf die Vielzahl von Elektrolyseur-Stacks (202, 204, 206) zum Sammeln des ersten Reaktionsgases des ersten Gasstroms (230) konfiguriert ist.

15. Gerät zum Betrieb eines Elektrolyseur-Stacks, umfassend:
einen Stromdichtemessung-Eingang, der so konfiguriert ist, dass er Stromdichte-Messwerte von jedem einer Vielzahl von Elektrolyseur-Stacks (202, 204, 206) empfängt;
ein Schalter-Steuergerät, das so konfiguriert ist, dass es mit Schaltern (203, 205, 207) jedes der mehreren Elektrolyseur-Stacks (202, 204, 206) gekoppelt werden kann, um einen beliebigen Elektrolyseur-Stack der Vielzahl der Elektrolyseur-Stacks (202, 204, 206) von der elektrischen Energie-Versorgung zu trennen;
eine Trigger-Signal-Schnittstelle und/oder eine Schnittstelle für einen gasempfindlichen Sensor; und
ein Steuergerät, das insbesondere einen Computer umfasst, welches
mit dem Stromdichtemessung-Eingang signalmäßig gekoppelt ist; und
mit dem Schalter-Steuergerät signalmäßig gekoppelt ist; und
mit der ersten Signal-Schnittstelle und/oder der Schnittstelle für den gasempfindlichen Sensor signalmäßig gekoppelt ist; und
wobei das Steuergerät so konfiguriert ist, dass es ein Verfahren gemäß Anspruch 1 bis 14 durchführen kann.

## Revendications

1. Procédé de fonctionnement d'une pluralité d'empilements d'électrolyseurs (202, 204, 206), chaque empilement de la pluralité d'empilements d'électrolyseurs (202, 204, 206) étant conçu pour être approvisionné en eau et en énergie électrique pour produire par voie électrochimique au moins un premier gaz de réaction au niveau d'un premier type d'électrode de chacun des empilements d'électrolyseurs (202, 204, 206), et le premier gaz de réaction produit par chaque empilement de la pluralité d'empilements d'électrolyseurs (202, 204, 206) étant combiné en un premier courant gazeux (230), comprenant :
la détermination d'une concentration d'impuretés, qui sont issues d'un deuxième gaz de réaction produit par voie électrochimique au niveau d'un deuxième type d'électrode de chacun des empilements d'électrolyseurs, à l'intérieur du premier courant gazeux (230) ;
le procédé étant **caractérisé par** les étapes suivantes :
génération d'un signal de déclenchement si la concentration des impuretés du deuxième gaz de réaction à l'intérieur du premier courant gazeux combiné (230) dépasse un niveau spécifique de deuxième gaz de réaction (104) ;
identification d'au moins un empilement d'électrolyseurs parmi la pluralité d'empilements d'électrolyseurs (202, 204, 206) qui est peu performant de par l'introduction excessive d'impuretés du deuxième gaz de réaction dans le premier courant gazeux (230), par mesure d'une densité de courant d'au moins un empilement d'électrolyseurs de la pluralité d'empilements d'électrolyseurs (202, 204, 206), si le signal de déclenchement est généré.

2. Procédé selon la revendication 1, dans lequel le premier gaz de réaction produit par voie électrochimique au niveau du premier type d'électrode est l'oxygène et le deuxième gaz de réaction produit par voie électrochimique au niveau du deuxième type d'électrode est l'hydrogène ; ou dans lequel le premier gaz de réaction produit par voie électrochimique au niveau du premier type d'électrode est l'hydrogène et le deuxième gaz de réaction produit par voie électrochimique au niveau du deuxième type d'électrode est l'oxygène.

3. Procédé selon l'une quelconque des revendications précédentes, dans lequel l'identification de l'au moins un empilement d'électrolyseurs peu performant parmi la pluralité d'empilements d'électrolyseurs (202, 204, 206) est réalisée par comparaison de la densité de courant mesurée de l'au moins un empilement d'électrolyseurs avec une densité de courant critique (104) ; et en particulier identifier l'au moins un empilement d'électrolyseurs peu performant si la densité de courant mesurée est inférieure à la densité de courant critique (104).

4. Procédé selon la revendication 2 ou 3, dans lequel le niveau spécifique de deuxième gaz de réaction est un niveau d'hydrogène gazeux de réaction si le premier gaz de réaction est l'oxygène ; et dans lequel le niveau spécifique de deuxième gaz de réaction est un niveau d'oxygène gazeux de réaction si le premier gaz de réaction est l'hydrogène ; et en particulier dans lequel le niveau d'hydrogène gazeux de réaction et le niveau d'oxygène gazeux de réaction sont différents.

5. Procédé selon l'une quelconque des revendications 3 et 4, dans lequel une densité de courant de chacun de plusieurs empilements donnés de la pluralité d'empilements d'électrolyseurs (202, 204, 206) est mesurée et chacune des densités de courant mesurées est comparée à une densité de courant critique individuelle, qui est assignée à chacun des empilements d'électrolyseurs donnés, pour identifier un empilement d'électrolyseurs peu performant individuel avec une densité de courant mesurée qui est la plus fortement inférieure à sa densité de courant critique individuelle, pour identifier l'empilement d'électrolyseurs peu performant.

6. Procédé selon l'une quelconque des revendications 3 et 5, dans lequel la densité de courant critique est actualisée pendant le fonctionnement de la pluralité d'empilements d'électrolyseurs (202, 204, 206) pour tenir compte de la dégradation des empilements d'électrolyseurs.

7. Procédé selon la revendication 5, dans lequel la densité de courant critique individuelle pour chacun des empilements d'électrolyseurs donnés est actualisée pendant le fonctionnement de ces empilements d'électrolyseurs pour tenir compte de la dégradation de ces empilements d'électrolyseurs.

8. Procédé selon la revendication 6 ou 7, dans lequel la densité de courant critique actualisée et/ou la densité de courant critique individuelle actualisée des empilements d'électrolyseurs sont déterminées pendant le fonctionnement de la pluralité d'empilements d'électrolyseurs par fonctionnement d'au moins certains empilements d'électrolyseurs de la pluralité d'empilements d'électrolyseurs à différentes consignes.

9. Procédé selon l'une quelconque des revendications 6 à 8, dans lequel la densité de courant critique actualisée et/ou la densité de courant critique individuelle actualisée des empilements d'électrolyseurs sont déterminées par mesure spectroscopique de l'impédance d'au moins plusieurs empilements d'électrolyseurs de la pluralité d'empilements d'électrolyseurs (202, 204, 206).

10. Procédé selon la revendication 9, dans lequel la mesure spectroscopique de l'impédance est effectuée au moyen d'harmoniques injectées par l'électricité fournie à la pluralité d'empilements d'électrolyseurs.

11. Procédé selon l'une quelconque des revendications précédentes, dans lequel l'empilement d'électrolyseurs peu performant identifié est débranché de son énergie électrique fournie.

12. Procédé selon la revendication 5 et la revendication 11, qui est répété jusqu'à ce que le signal de déclenchement ne soit plus généré.

13. Procédé selon l'une quelconque des revendications précédentes, dans lequel la concentration d'impuretés du deuxième gaz de réaction est déterminée par un capteur sensible aux gaz pour générer le signal de déclenchement.

14. Procédé selon la revendication 13, dans lequel le capteur sensible aux gaz est positionné pour la détection à l'intérieur du premier courant gazeux (230) ; ou dans lequel le capteur sensible aux gaz est positionné pour la détection à l'intérieur d'un réservoir (220), le réservoir (220) étant conçu par rapport à la pluralité d'empilements d'électrolyseurs (202, 204, 206) pour collecter le premier gaz de réaction du premier courant gazeux (230).

15. Dispositif de fonctionnement d'empilements d'électrolyseurs, comprenant :
une entrée de mesure de densité de courant, qui est conçue pour recevoir des valeurs de mesure de densité de courant de chaque empilement d'une pluralité d'empilements d'électrolyseurs (202, 204, 206) ;
un dispositif de commande de commutation, qui est conçu pour être couplé par signaux à des commutateurs (203, 205, 207) de chaque empilement de la pluralité d'empilements d'électrolyseurs (202, 204, 206), en vue de débrancher n'importe quel empilement d'électrolyseurs de la pluralité d'empilements d'électrolyseurs (202, 204, 206) de l'énergie électrique fournie ;
une interface de signal de déclenchement et/ou une interface pour un capteur sensible aux gaz ; et
un dispositif de commande, comportant en particulier un ordinateur, qui est couplé par signaux à l'entrée de mesure de densité de courant ; et couplé par signaux au dispositif de commande de commutation ; et couplé par signaux à la première interface de signal et/ou l'interface pour le capteur sensible aux gaz ; et dans lequel le dispositif de commande est conçu pour effectuer un procédé selon les revendications 1 à 14.
